# EUROPEAN PATENT SPECIFICATION

(11) **EP 2 936 544 B1**
(45) Date of publication and mention of the grant of the patent: **28.02.2024**
(21) Application number: 13865198.9
(22) Date of filing: 21.11.2013
(51) Int. Cl.: G16C 20/20, H01J 49/26, H01J 49/00

(54) **COMPOUND IDENTIFICATION USING MULTIPLE SPECTRA AT DIFFERENT COLLISION ENERGIES**
IDENTIFIZIERUNG EINER VERBINDUNG MIT MEHREREN SPEKTREN BEI VERSCHIEDENEN KOLLISIONSENERGIEN
IDENTIFICATION D'UN COMPOSÉ EN UTILISANT DE MULTIPLES SPECTRES À DES ÉNERGIES DE COLLISION DIFFÉRENTES

(30) Priority: 20.12.2012 US 201261740369 P
(43) Date of publication of application: 28.10.2015
(73) Proprietor: DH Technologies Development Pte. Ltd., Singapore 739256 (SG)
(72) Inventor: COX, David, M., North York, Ontario M2N 2C4 (CA)
(74) Representative: J A Kemp LLP
(86) International application number: PCT/IB2013/002607
(87) International publication number: WO 2014/096915

(56) References cited:
- EP-A1- 2 450 815
- EP-A2- 1 457 776
- WO-A1-2012/111249
- WO-A2-2009/138179
- US-A1- 2006 003 385
- US-A1- 2010 179 766
- US-A1- 2012 241 603
- RICHARD W. VACHET ET AL: "Correlation of Kinetic Energy Losses in High-Energy Collision-Induced Dissociation with Observed Peptide Product Ions", ANALYTICAL CHEMISTRY, vol. 68, no. 3, 1 January 1996 (1996-01-01), pages 522-526, XP055282416, ISSN: 0003-2700, DOI: 10.1021/ac950893r
- MARCH ET AL: "High-energy and low-energy collision-induced dissociation of protonated flavonoids generated by MALDI and by electrospray ionization", INTERNATIONAL JOURNAL OF MASS SPECTROMETRY, ELSEVIER SCIENCE PUBLISHERS, AMSTERDAM, NL, vol. 262, no. 1-2, 23 February 2007 (2007-02-23), pages 51-66, XP005903429, ISSN: 1387-3806, DOI: 10.1016/J.IJMS.2006.10.008
- CLAEYS M ET AL: "Comparison of high- and low-energy collision-induced dissociation tandem mass spectrometry in the analysis of glycoalkaloids and their aglycons", JOURNAL OF THE AMERICAN SOCIETY FOR MASS SPECTROMETRY, ELSEVIER SCIENCE INC, US, vol. 7, no. 2, 1 February 1996 (1996-02-01), pages 173-181, XP004720359, ISSN: 1044-0305, DOI: 10.1016/1044-0305(95)00636-2
- PAPAYANNOPOULOS I A: "THE INTERPRETATION OF COLLISION-INDUCED DISSOCIATION TANDEM MASS SPECTRA OF PEPTIDES", MASS SPECTROMETRY REVIEWS, JOHN WILEY AND SONS, NEW YORK, NY, US, vol. 14, 1 January 1995 (1995-01-01), pages 49-73, XP001051562, ISSN: 0277-7037, DOI: 10.1002/MAS.1280140104
- Tamar Geiger ET AL: "Proteomics on an Orbitrap benchtop mass spectrometer using all-ion fragmentation", Molecular & cellular proteomics : MCP, 1 October 2010 (2010-10-01), pages 2252-2261, XP055139171, United States DOI: 10.1074/mcp.M110.001537 Retrieved from the Internet: URL:http://www.ncbi.nlm.nih.gov/pubmed/206 10777
- Jason C. Rogalski ET AL: "Statistical evaluation of electrospray tandem mass spectra for optimized peptide fragmentation", JOURNAL OF THE AMERICAN SOCIETY FOR MASS SPECTROMETRY., vol. 16, no. 4, 1 April 2005 (2005-04-01), pages 505-514, XP55547890, US ISSN: 1044-0305, DOI: 10.1016/j.jasms.2005.01.002

## Description

The present invention relates to systems and methods for compound identification.

Identifying a compound from tandem mass spectrometry, or mass spectrometry/mass spectrometry (MS/MS), spectra is often ambiguous. The existing algorithms (dot product, probability based, etc.) give a score representing the similarity between the acquired spectra and the library spectra. However, it is difficult to know what a good score is. In other words, it is difficult to know what score will confidently identify a compound.

Some compounds have very few, or no, distinguishing fragments, which result in scores that do not represent the confidence in the identification. For example, a compound with no fragments will get a very high Fit and Purity score, but so will many other compounds that have poor fragmentation. This makes it hard to distinguish false positives from a true hit.

In other cases, a moderately rich fragmentation pattern has interference from a co-eluting compound, which adversely affects the score. This makes it difficult to know what score is appropriate to confidently identify the compound.

Historically, library searching was most often used on gas chromatography coupled mass spectrometry (GC-MS) systems electron impact (EI) sources. These systems and sources had a very well defined energy, leading to very reproducible spectra among different laboratories and even across instrument models. For liquid chromatography coupled tandem mass spectrometry (LC-MS/MS) spectra, the fragmentation pattern depends heavily on the collision energy (CE) used and can vary somewhat between instruments. This makes it difficult to set appropriate scores for identifying a compound across different laboratories.

WO 2009/138179 A2, EP 1457776 A2, Tamar Geiger ET AL: "Proteomics on an Orbitrap benchtop mass spectrometer using all-ion fragmentation", Molecular & cellular proteomics : MCP, 1 October 2010 (2010-10-01), pages 2252-2261 and Jason C. Rogalski ET AL: "Statistical evaluation of electrospray tandem mass spectra for optimized peptide fragmentation", JOURNAL OF THE AMERICAN SOCIETY FOR MASS SPECTROMETRY., vol. 16, no. 4, 1 April 2005 (2005-04-01), pages 505-514 disclose mass spectrometry methods.

The invention is defined in independent claims 1, 7 and 13. Further aspects and preferred embodiments are defined in the dependent claims. Any aspects, embodiments and examples of the present disclosure which do not fall under the scope of the appended claims do not form part of the invention and are merely provided for illustrative purposes.

A system is disclosed for compound identification using multiple spectra that are a function of collision energy that affects the intensity of fragment ions. A mass spectrometer analyzes a sample. Within each cycle of the analysis the mass spectrometer selects at least one ion and fragments that ion using two or more values for a collision energy that affects the intensity of fragment ions. A plurality of fragment ion spectra are produced that are a function of the collision energy.

A processor receives the plurality of acquired fragment ion spectra. The processor identifies the at least one ion by comparing rates of change of mass intensity, with respect to the collision energy, for acquired and known fragment ions. Specifically, one or more acquired rates of change calculated for acquired fragment ions from the plurality of acquired fragment ion spectra are compared with one or more known rates of change calculated for one or more stored fragment ions of one or more known compounds in a database of known compounds. The database of known compounds includes for each known compound a plurality of known fragment ion spectra that are also a function of the collision energy.

A method is disclosed for compound identification using multiple spectra that are a function of a collision energy that affects the intensity of fragment ions. A plurality of acquired fragment ion spectra that are a function of a variable collision energy for at least one ion are received from a mass spectrometer using a processor.

The at least one ion is identified by comparing rates of change of mass intensity, with respect to the collision energy, for acquired and known fragment ions using the processer. Specifically, one or more acquired rates of change calculated for acquired fragment ions from the plurality of acquired fragment ion spectra are compared with one or more known rates of change calculated for one or more stored fragment ions of one or more known compounds in a database of known compounds.

A computer program product is disclosed that includes a non-transitory and tangible computer-readable storage medium whose contents include a program with instructions being executed on a processor so as to perform a method for compound identification using multiple spectra that are a function of a collision energy that affects the intensity of fragment ions. In various embodiments, the method includes providing a system, wherein the system comprises one or more distinct software modules, and wherein the distinct software modules comprise a measurement module and an analysis module.

The measurement module receives a plurality of acquired fragment ion spectra that are a function of a collision energy for at least one ion from a mass spectrometer using the measurement module. The analysis module identifies the at least one ion by comparing rates of change of mass intensity, with respect to the collision energy, for acquired and known fragment ions. Specifically, one or more acquired rates of change calculated for acquired fragment ions from the plurality of acquired fragment ion spectra are compared with one or more known rates of change calculated for one or more stored fragment ions of one or more known compounds in a database of known compounds.

These and other features of the applicant's teachings are set forth herein.

The skilled artisan will understand that the drawings, described below, are for illustration purposes only. The drawings are not intended to limit the scope of the present teachings in any way.

Figure 1 is a block diagram that illustrates a computer system, upon which embodiments of the present teachings may be implemented.

Figure 2 is a schematic diagram showing a system for compound identification using multiple spectra.

Figure 3 is an exemplary flowchart showing a method for compound identification using multiple spectra that are a function of a variable instrument parameter that affects the intensity of fragment ions. The only variable instrument parameter in the scope of the invention is a collision energy.

Figure 4 is a schematic diagram of a system that includes one or more distinct software modules that performs a method for compound identification using multiple spectra that are a function of a collision energy that affects the intensity of fragment ions, in accordance with various embodiments.

Before one or more embodiments of the present teachings are described in detail, one skilled in the art will appreciate that the present teachings are not limited in their application to the details of construction, the arrangements of components, and the arrangement of steps set forth in the following detailed description or illustrated in the drawings.

### COMPUTER-IMPLEMENTED SYSTEM

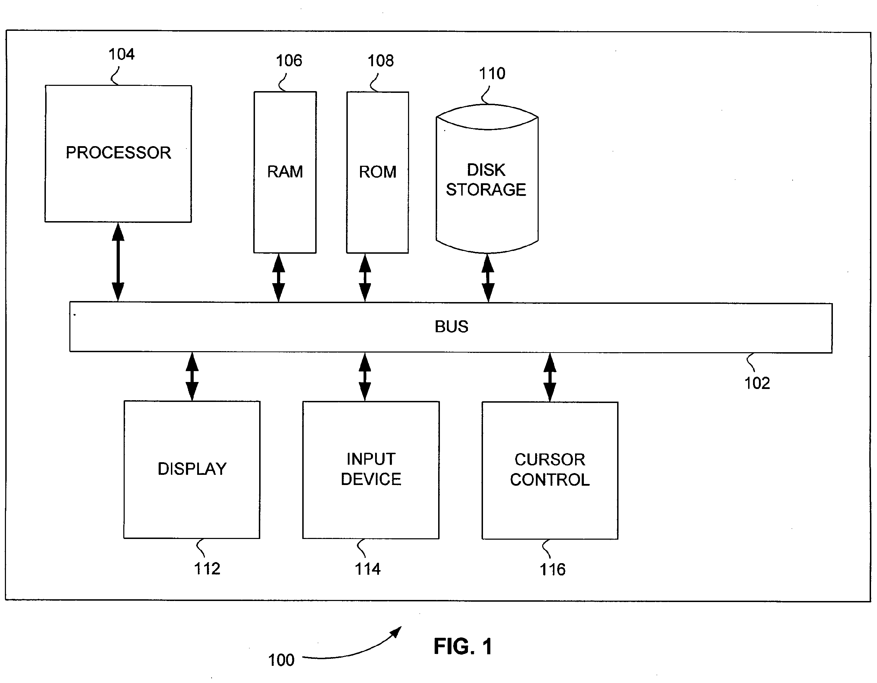

Figure 1 is a block diagram that illustrates a computer system 100, upon which embodiments of the present teachings may be implemented. Computer system 100 includes a bus 102 or other communication mechanism for communicating information, and a processor 104 coupled with bus 102 for processing information. Computer system 100 also includes a memory 106, which can be a random access memory (RAM) or other dynamic storage device, coupled to bus 102 for storing instructions to be executed by processor 104. Memory 106 also may be used for storing temporary variables or other intermediate information during execution of instructions to be executed by processor 104. Computer system 100 further includes a read only memory (ROM) 108 or other static storage device coupled to bus 102 for storing static information and instructions for processor 104. A storage device 110, such as a magnetic disk or optical disk, is provided and coupled to bus 102 for storing information and instructions.

Computer system 100 may be coupled via bus 102 to a display 112, such as a cathode ray tube (CRT) or liquid crystal display (LCD), for displaying information to a computer user. An input device 114, including alphanumeric and other keys, is coupled to bus 102 for communicating information and command selections to processor 104. Another type of user input device is cursor control 116, such as a mouse, a trackball or cursor direction keys for communicating direction information and command selections to processor 104 and for controlling cursor movement on display 112. This input device typically has two degrees of freedom in two axes, a first axis (*i.e.*, x) and a second axis (*i.e.*, y), that allows the device to specify positions in a plane.

A computer system 100 can perform the present teachings. Consistent with certain implementations of the present teachings, results are provided by computer system 100 in response to processor 104 executing one or more sequences of one or more instructions contained in memory 106. Such instructions may be read into memory 106 from another computer-readable medium, such as storage device 110. Execution of the sequences of instructions contained in memory 106 causes processor 104 to perform the process described herein. Alternatively hard-wired circuitry may be used in place of or in combination with software instructions to implement the present teachings. Thus implementations of the present teachings are not limited to any specific combination of hardware circuitry and software.

The term "computer-readable medium" as used herein refers to any media that participates in providing instructions to processor 104 for execution. Such a medium may take many forms, including but not limited to, non-volatile media, volatile media, and transmission media. Non-volatile media includes, for example, optical or magnetic disks, such as storage device 110. Volatile media includes dynamic memory, such as memory 106. Transmission media includes coaxial cables, copper wire, and fiber optics, including the wires that comprise bus 102.

Common forms of computer-readable media include, for example, a floppy disk, a flexible disk, hard disk, magnetic tape, or any other magnetic medium, a CD-ROM, digital video disc (DVD), a Blu-ray Disc, any other optical medium, a thumb drive, a memory card, a RAM, PROM, and EPROM, a FLASH-EPROM, any other memory chip or cartridge, or any other tangible medium from which a computer can read.

Various forms of computer readable media may be involved in carrying one or more sequences of one or more instructions to processor 104 for execution. For example, the instructions may initially be carried on the magnetic disk of a remote computer. The remote computer can load the instructions into its dynamic memory and send the instructions over a telephone line using a modem. A modem local to computer system 100 can receive the data on the telephone line and use an infra-red transmitter to convert the data to an infra-red signal. An infra-red detector coupled to bus 102 can receive the data carried in the infra-red signal and place the data on bus 102. Bus 102 carries the data to memory 106, from which processor 104 retrieves and executes the instructions. The instructions received by memory 106 may optionally be stored on storage device 110 either before or after execution by processor 104.

In accordance with various embodiments, instructions configured to be executed by a processor to perform a method are stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

The following descriptions of various implementations of the present teachings have been presented for purposes of illustration and description. It is not exhaustive and does not limit the present teachings to the precise form disclosed. Modifications and variations are possible in light of the above teachings or may be acquired from practicing of the present teachings. Additionally, the described implementation includes software but the present teachings may be implemented as a combination of hardware and software or in hardware alone. The present teachings may be implemented with both object-oriented and non-object-oriented programming systems.

### SYSTEMS AND METHODS FOR COMPOUND IDENTIFICATION

As described above, trying to identify a compound from tandem mass spectrometry spectra can often produce ambiguous results. This can be due to the difficulty in scoring comparisons of the acquired spectra to a library or database of stored spectra for known compounds. In addition, some tandem mass spectrometry methods, such as liquid chromatography coupled tandem mass spectrometry (LC-MS/MS), produce spectra that depend heavily on a variable instrument parameter, such as collision energy (CE). As a result, it is difficult to compare the results from one instrument across different instruments or laboratories.

In various embodiments, a compound is identified by comparing the rate of change of one or more acquired mass intensities, with respect to a collision energy to the rate of change of one or more database stored mass intensities, with respect to the same variable instrument parameter. One skilled in the art can appreciate that a mass intensity can also include a mass-to-charge ratio (m/z) intensity.

Comparing rates of change of mass intensity with respect to a variable instrument parameter makes the process of identifying a compound less ambiguous. Ambiguity is reduced because a rate of change contains more information. It contains information from at least two measurements of the mass intensity. Conventionally, a comparison of the mass intensity, or a determination of whether or not the mass is there, is based on just one measurement of the mass intensity.

Also, using rates of change of mass intensity to identify compounds improves the consistency of results across different instruments or laboratories. Different instruments or laboratories can measure different mass intensity absolute values for a compound of interest, if they use different values for the variable instrument parameter. These mass intensity absolute values for the compound of interest can also vary from the mass intensity absolute values stored in the database of spectra for known compounds.

However, as long as the different instruments or laboratories produce mass intensity values that share a common range of values for the variable instrument parameter with the database of spectra for known compounds that is searched, the results will be consistent. This is because the rate of change of mass intensity values for a compound with respect to a common range of values for a variable instrument parameter will be similar across all instruments, laboratories, and the database of spectra for known compounds for the common range. This is true even though the mass intensity absolute values across those instruments, laboratories, and the database of spectra for known compounds compound may be different at specific values over the common range.

In various embodiments, collision energy (CE) is the variable instrument parameter that is used to identify a compound from tandem mass spectrometry spectra. CE is a parameter that affects the intensity of fragment ions. A fast tandem mass spectrometer can perform multiple fragmentation ion scans, or product ion scans, within a single cycle time of the instrument. As a result, a compound of interest, or precursor ion, can be fragmented using several different CEs per cycle, producing a plurality of product ion spectra per cycle. The combined set of product ion spectra provide information on how the mass intensity of each fragment or product ion changes with respect to CE.

A rate of change of the mass intensity of each product ion with respect to CE is calculated. The rate of change of the mass intensity of one or more product ions with respect to CE can be used to identify the precursor ion. The rate or rates of change calculated for the one or more product ions is compared to the rate or rates of change of product ions of known compounds.

Some conventional libraries or databases of known compounds include spectra for product ions that were collected using different collision energies. Such conventional libraries are used to compare data from different instruments that may have been collected at different CEs. As a result, they are designed for comparing one spectrum at a time. In other words, they do not readily provide a rate of change of a product ion with respect to the CE. However, all the data is inherent in such a database. Consequently, even some conventional libraries can be used to identify a compound from a rate of change, if a rate of change for one or more product ion the libraries is also calculated.

The rate of change of mass intensity with respect to CE, or mass intensity as function of CE, of an acquired product ion can be compared to the mass intensity as function of CE of a stored product ion of a library of spectra of known compounds in a variety of different ways. For example, the mass intensity versus CE can be plotted for every product ion, and the shape of these breakdown curves can be compared or measured against library data.

In another embodiment, the acquired data can be converted to an image. Image comparison tools are then used to score the match. Elements of an image, for example, are a combination of the mass, mass intensity, and CE. The extra dimension of CE makes it significantly easier to extract multiple compound identifications. For example, using common image matching algorithms (cross correlation matching) one can confidently identify one compound, remove these features from the image, and then identify other compounds.

Although the examples herein describe systems and methods that identify a compound from tandem mass spectrometer data by using the rate of change of mass intensity as a function of CE, various embodiments are not limited to tandem mass spectrometry.

### System for Compound Identification

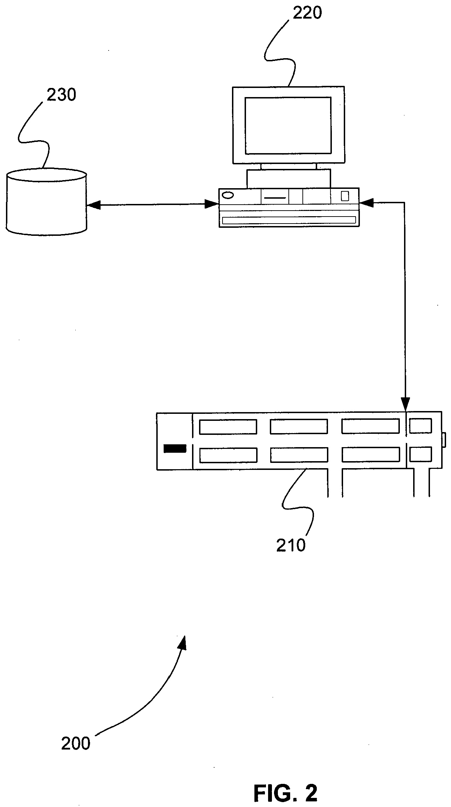

Figure 2 is a schematic diagram showing a system 200 for compound identification using multiple spectra that are a function of a variable instrument parameter that affects the intensity of fragment ions, in accordance with various embodiments. System 200 includes mass spectrometer 210, processor 220, and database 230.

Mass spectrometer 210 can include one or more physical mass analyzers that perform one or more mass analyses. A mass analyzer of a tandem mass spectrometer can include , but is not limited to, a time-of-flight (TOF), quadrupole, an ion trap, a linear ion trap, an orbitrap, or a Fourier transform mass analyzer. Mass spectrometer 210 can also include a one or more separation devices (not shown). The separation device can perform a separation technique that includes, but is not limited to, liquid chromatography, gas chromatography, capillary electrophoresis, or ion mobility. Mass spectrometer 210 can include separating mass spectrometry stages or steps in space or time, respectively.

Processor 220 can be, but is not limited to, a computer, microprocessor, or any device capable of sending and receiving control signals and data to and from mass spectrometer 210 and processing data. Processor 220 is in communication with mass spectrometer 210.

Database 230 can include magnetic or electronic storage. Database 230 can be part of a memory for processor 220 or it can be a separate memory. Database 230 can include software components in addition to hardware components.

Mass spectrometer 210 analyzes a sample. Within each cycle of the analysis, mass spectrometer 210 selects at least one ion and fragments that ion using two or more values for a variable instrument parameter that affects the intensity of fragment ions. A plurality of fragment ion spectra are produced that are a function of the variable instrument parameter.

Mass spectrometer 210 analyzes the sample using mass spectrometry/mass spectrometry (MS/MS), for example. In various alternative embodiments, spectrometer 210 analyzes the sample using mass spectrometry/mass spectrometry/mass spectrometry (MS³), for example. The variable instrument parameter is collision energy (CE).

Database 230 stores fragment ion spectra that are a function of variable the variable instrument parameter for known compounds. Database 230 includes for each fragment ion of each known compound a plurality of fragment ion spectra that are a function of the variable instrument parameter.

Processor 220 receives the plurality of acquired fragment ion spectra for the at least one ion from mass spectrometer 210. Processor 220 receives the plurality of acquired fragment ion spectra in a post-acquisition step, for example. Processor 220 identifies the at least one ion by comparing the rates of change of mass intensity with respect to the variable instrument parameter of acquired and known fragments ions. More specifically, processor 220 compares one or more acquired rates of change of mass intensity, with respect to the variable instrument parameter, calculated for one or more acquired fragment ions from the plurality of acquired fragment ion spectra with one or more known rates of change of mass intensity, with respect to the variable instrument parameter, calculated for one or more stored fragment ions of one or more known compounds in database 230.

In various embodiments, processor 220 identifies the at least one ion by scoring the comparison of the rates of change of acquired and known fragment ions, calculating scores for a list of known compounds based on the scores of the comparison, and selecting a known compound from the list.

In various embodiments, comparing the rates of change involves comparing acquired and known breakdown curves. More specifically, one or more acquired breakdown curves of mass intensity versus the collision energy parameter calculated for one or more acquired fragment ions from the plurality of acquired fragment ion spectra are compared with one or more known breakdown curves of mass intensity versus the variable instrument parameter calculated for one or more stored fragment ions of one or more known compounds in database 230.

In various embodiments, comparing the rates of change involves comparing acquired and known images of fragment ion data. Each element of each image is, for example, a combination of mass, mass intensity, and collision energy (CE). More specifically, one or more acquired images calculated for one or more acquired fragment ions from the plurality of acquired fragment ion spectra are compared with one or more known images calculated for one or more stored fragment ions of one or more known compounds in database 230.

### Method for Compound Identification

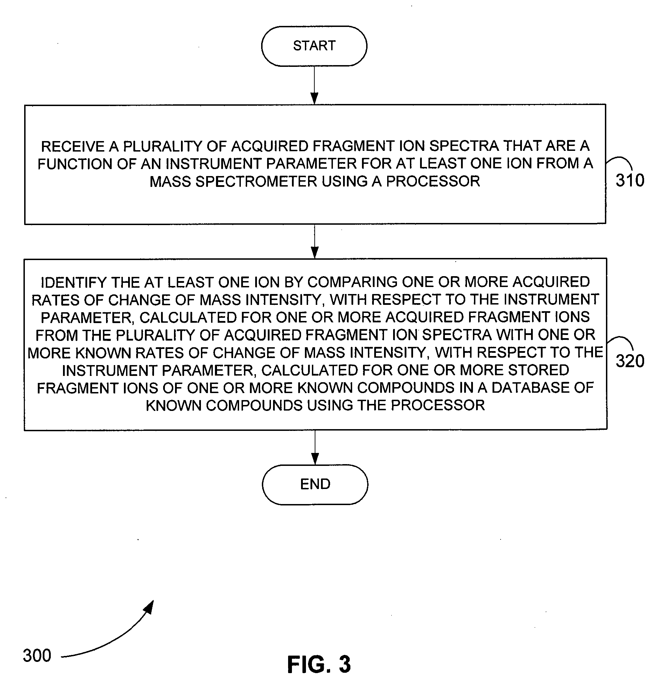

Figure 3 is an exemplary flowchart showing a method 300 for compound identification using multiple spectra that are a function of a variable instrument parameter that affects the intensity of fragment ions, in accordance with various embodiments.

In step 310 of method 300, a plurality of acquired fragment ion spectra that are a function of a variable instrument parameter for at least one ion are received from a mass spectrometer using a processor. The plurality of acquired fragment ion spectra are produced from an analysis of a sample by the mass spectrometer. Within each cycle of the analysis the mass spectrometer selects the at least one ion and fragments the at least one ion using two or more values for the variable instrument parameter that affects the intensity of fragment ions, producing the plurality of acquired fragment ion spectra.

In step 320, the at least one ion is identified by comparing rates of change of mass intensity, with respect to the variable instrument parameter, for acquired and known fragment ions using the processor. One or more acquired rates of change calculated for acquired fragment ions from the plurality of acquired fragment ion spectra are compared with one or more known rates of change calculated for one or more stored fragment ions of one or more known compounds in a database of known compounds. The database of known compounds includes for each fragment ion of each known compound a plurality of known fragment ion spectra that are also a function of the variable instrument parameter. The only instrument parameter in the scope of the invention is a collision energy.

### Computer Program Product for Compound Identification

In various embodiments, computer program products include a tangible computer-readable storage medium whose contents include a program with instructions being executed on a processor so as to perform a method for compound identification using multiple spectra that are a function of a collision energy that affects the intensity of fragment ions. This method is performed by a system that includes one or more distinct software modules.

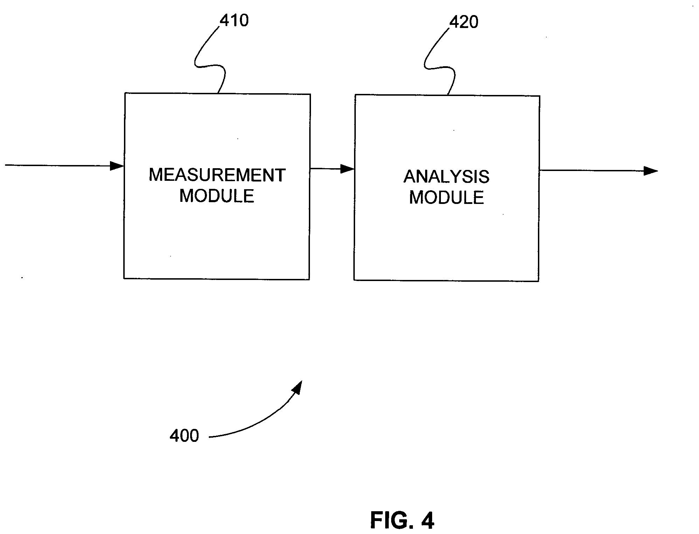

Figure 4 is a schematic diagram of a system 400 that includes one or more distinct software modules that performs a method for compound identification using multiple spectra that are a function of a collision energy that affects the intensity of fragment ions, in accordance with various embodiments. System 400 includes measurement module 410 and analysis module 420.

Measurement module 410 receives a plurality of acquired fragment ion spectra that are a function of a variable instrument parameter for at least one ion from a mass spectrometer using the measurement module. The plurality of acquired fragment ion spectra are produced from an analysis of a sample by the mass spectrometer. Within each cycle of the analysis the mass spectrometer selects the at least one ion and fragments the at least one ion using two or more values for the variable instrument parameter that affects the intensity of fragment ions, producing the plurality of acquired fragment ion spectra.

Analysis module 420 identifies the at least one ion by comparing rates of change of mass intensity, with respect to the collision energy, for acquired and known fragment ions. One or more acquired rates of change calculated for acquired fragment ions from the plurality of acquired fragment ion spectra are compared with one or more known rates of change calculated for one or more stored fragment ions of one or more known compounds in a database of known compounds. The database of known compounds includes for each fragment ion of each known compound a plurality of known fragment ion spectra that are also a function of the collision energy.

While the present teachings are described in conjunction with various embodiments, it is not intended that the present teachings be limited to such embodiments.

Further, in describing various embodiments, the specification may have presented a method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. In addition, the claims directed to the method and/or process should not be limited to the performance of their steps in the order written, and one skilled in the art can readily appreciate that the sequences may be varied and still remain within the scope of the various embodiments. The scope is defined in the claims.

## Claims

1. A system (200) for compound identification using multiple spectra that are a function of collision energy, comprising:
a mass spectrometer (210) that is configured to analyze a sample and within each cycle of the mass spectrometer to select at least one ion and to fragment the at least one ion using two or more collision energies, producing a plurality of acquired fragment ion spectra that are a function of collision energy;
a database (230) of known compounds that includes for each known compound a plurality of fragment ion spectra that are a function of collision energy; and
a processor (220) in communication with the mass spectrometer and the database that is configured to:
receive the plurality of acquired fragment ion spectra for the at least one ion from the mass spectrometer,
calculate one or more sample fragment ion rates of change of mass intensity, with respect to collision energy, for one or more sample fragment ions from the plurality of acquired fragment ion spectra
calculate one or more database fragment ion rates of change of mass intensity, with respect to collision energy, for one or more database fragment ions of one or more known compounds in the database, and
compare the one or more sample fragment ion rates of change of mass intensity with the one or more database fragment ion rates of change of mass intensity to identify the at least one ion.

2. The system (200) of claim 1, wherein the mass spectrometer (210) is configured to analyze the sample using tandem mass spectrometry, or mass spectrometry/mass spectrometry (MS/MS).

3. The system (200) of claim 1, wherein the mass spectrometer (210) is configured to analyze the sample using mass spectrometry/mass spectrometry/mass spectrometry (MS³).

4. The system (200) of any one of claims 1 to 3, wherein comparing the one or more sample fragment ion rates of change with the one or more database fragment ion rates of change comprises
comparing one or more acquired curves of mass intensity versus collision energy calculated for one or more acquired fragment ions from the plurality of acquired fragment ion spectra with one or more known curves of mass intensity versus collision energy calculated for one or more stored fragment ions of one or more known compounds in the database.

5. The system (200) of any one of claims 1 to 3, wherein comparing the one or more sample fragment ion rates of change with the one or more database fragment ion rates of change comprises
comparing one or more acquired images calculated for one or more acquired fragment ions from the plurality of acquired fragment ion spectra with one or more known images calculated for one or more stored fragment ions of one or more known compounds in the database.

6. The system (200) of claim 5, wherein each element of each image of the one or more acquired images is a combination of mass, mass intensity, and collision energy (CE) and each element of each image of the one or more known images is a combination of mass, mass intensity, and collision energy (CE).

7. A method (300) for compound identification using multiple spectra that are a function of collision energy, comprising:
receiving (310) a plurality of acquired fragment ion spectra that are a function of collision energy for at least one ion from a mass spectrometer using a processor, wherein the mass spectrometer analyzes a sample and within each cycle of the mass spectrometer selects the at least one ion and fragments the at least one ion using two or more collision energies, producing the plurality of acquired fragment ion spectra;
calculating one or more sample fragment ion rates of change of mass intensity, with respect to collision energy, for one or more sample fragment ions from the plurality of acquired fragment ion spectra;
calculating one or more database fragment ion rates of change of mass intensity, with respect to collision energy, for one or more database fragment ions of one or more known compounds in the database, wherein the database of known compounds includes for each fragment ion of each known compound a plurality of fragment ion spectra that are a function of the collision energy; and
comparing (320) the one or more sample fragment ion rates of change of mass intensity with the one or more database fragment ion rates of change of mass intensity to identify the at least one ion.

8. The method (300) of claim 7, wherein the mass spectrometer analyzes the sample using tandem mass spectrometry, or mass spectrometry/mass spectrometry (MS/MS).

9. The method (300) of claim 7, wherein the mass spectrometer analyzes the sample using mass spectrometry/mass spectrometry/mass spectrometry (MS³).

10. The method (300) of any one of claims 7 to 9, wherein comparing the one or more sample fragment ion rates of change with the one or more database fragment ion rates of change comprises
comparing one or more acquired curves of mass intensity versus collision energy calculated for one or more acquired fragment ions from the plurality of acquired fragment ion spectra with one or more known curves of mass intensity versus collision energy calculated for one or more stored fragment ions of one or more known compounds in the database.

11. The method (300) of any one of claims 7 to 9, wherein comparing the one or more sample fragment ion rates of change with the one or more database fragment ion rates of change comprises
comparing one or more acquired images calculated for one or more acquired fragment ions from the plurality of acquired fragment ion spectra with one or more known images calculated for one or more stored fragment ions of one or more known compounds in the database.

12. The method (300) of claim 11, wherein each element of each image of the one or more sample fragment ion images is a combination of mass, mass intensity, and collision energy (CE) and each element of each image of the one or more database fragment ion images is a combination of mass, mass intensity, and collision energy (CE).

13. A computer program product, comprising a non-transitory and tangible computer-readable storage medium whose contents include a program with instructions being executed on a processor so as to perform a method for compound identification using multiple spectra that are a function of collision energy, the method comprising:
providing a system (400), wherein the system comprises one or more distinct software modules, and wherein the distinct software modules comprise a measurement module (410) and an analysis module (420);
receiving (310) a plurality of acquired fragment ion spectra that are a function of collision energy for at least one ion from a mass spectrometer using the measurement module, wherein the mass spectrometer analyzes a sample and within each cycle of the mass spectrometer selects the at least one ion and fragments the at least one ion using two or more collision energies, producing the plurality of acquired fragment ion spectra;
calculating one or more sample fragment ion rates of change of mass intensity, with respect to collision energy, for one or more sample fragment ions from the plurality of acquired fragment ion spectra;
calculating one or more database fragment ion rates of change of mass intensity, with respect to collision energy, for one or more database fragment ions of one or more known compounds in the database, wherein the database of known compounds includes for each fragment ion of each known compound a plurality of fragment ion spectra that are a function of collision energy; and
comparing (320) the one or more sample fragment ion rates of change of mass intensity with the one or more database fragment ion rates of change of mass intensity to identify the at least one ion.

## Patentansprüche

1. System (200) zur Verbindungsidentifikation unter Verwendung von mehreren Spektren, die eine Funktion von Kollisionsenergie sind, umfassend:
ein Massenspektrometer (210), das konfiguriert ist, um eine Probe zu analysieren und um in jedem Zyklus des Massenspektrometers mindestens ein Ion auszuwählen und um das mindestens eine Ion unter Verwendung von zwei oder mehreren Kollisionsenergien zu fragmentieren, wodurch eine Vielzahl von erfassten Fragmentionenspektren erzeugt wird, die eine Funktion von Kollisionsenergie sind;
eine Datenbank (230) aus bekannten Verbindungen, die für jede bekannte Verbindung eine Vielzahl von Fragmentionenspektren beinhaltet, die eine Funktion von Kollisionsenergie sind; und
einen Prozessor (220) in Kommunikation mit dem Massenspektrometer und der Datenbank, der für Folgendes konfiguriert ist:
Empfangen der Vielzahl von erfassten Fragmentionenspektren für das mindestens eine Ion aus dem Massenspektrometer,
Berechnen von einer oder mehreren Probefragmentionenraten der Veränderung von Massenintensität in Bezug auf Kollisionsenergie für ein oder mehrere Probefragmentionen aus der Vielzahl von erfassten Fragmentionenspektren
Berechnen von einer oder mehreren Datenbankfragmentionenraten der Veränderung von Massenintensität in Bezug auf Kollisionsenergie für ein oder mehrere Datenbankfragmentionen aus einer oder mehreren bekannten Verbindungen in der Datenbank und
Vergleichen der einen oder mehreren Probefragmentionenraten der Veränderung von Massenintensität mit der einen oder den mehreren Datenbankfragmentionenraten der Veränderung von Massenintensität, um das mindestens eine Ion zu identifizieren.

2. System (200) nach Anspruch 1, wobei das Massenspektrometer (210) konfiguriert ist, um die Probe unter Verwendung von Tandem-Massenspektrometrie oder Massenspektrometrie/Massenspektrometrie (MS/MS) zu analysieren.

3. System (200) nach Anspruch 1, wobei das Massenspektrometer (210) konfiguriert ist, um die Probe unter Verwendung von Massenspektrometrie/Massenspektrometrie/Massenspektrometrie (MS³) zu analysieren.

4. System (200) nach einem der Ansprüche 1 bis 3, wobei das Vergleichen der einen oder mehreren Probefragmentionenraten der Veränderung mit der einen oder den mehreren Datenbankfragmentionenraten der Veränderung Folgendes umfasst
Vergleichen von einer oder mehreren erfassten Kurven von Massenintensität im Vergleich zu Kollisionsenergie, berechnet für ein oder mehrere erfasste Fragmentionen aus der Vielzahl von erfassten Framentionenspektren mit einer oder mehreren bekannten Kurven von Massenintensität im Vergleich zu Kollisionsenergie, berechnet für ein oder mehrere gespeicherte Fragmentionen von einer oder mehreren bekannten Verbindungen in der Datenbank.

5. System (200) nach einem der Ansprüche 1 bis 3, wobei das Vergleichen der einen oder mehreren Probefragmentionenraten der Veränderung mit einer oder mehreren Datenbankfragmentionenraten der Veränderung Folgendes umfasst
Vergleichen von einem oder mehreren erfassten Bildern, berechnet für ein oder mehrere erfasste Fragmentionen aus der Vielzahl von erfassten Fragmentionenspektren mit einem oder mehreren bekannten Bildern, berechnet für ein oder mehrere gespeicherte Fragmentionen von einer oder mehreren bekannten Verbindungen in der Datenbank.

6. System (200) nach Anspruch 5, wobei jedes Element von jedem Bild aus dem einen oder den mehreren erfassten Bildern eine Kombination aus Masse, Massenintensität und Kollisionsenergie (CE) ist, und jedes Element von jedem Bild aus dem einen oder den mehreren bekannten Bildern eine Kombination aus Masse, Massenintensität und Kollisionsenergie (CE) ist.

7. Verfahren (300) zur Verbindungsidentifikation unter Verwendung von mehreren Spektren, die eine Funktion von Kollisionsenergie sind, umfassend:
Empfangen (310) einer Vielzahl von erfassten Fragmentionenspektren, die eine Funktion von Kollisionsenergie sind, für mindestens ein Ion aus einem Massenspektrometer, unter Verwendung eines Prozessors, wobei das Massenspektrometer eine Probe analysiert und in jedem Zyklus des Massenspektrometers das mindestens eine Ion auswählt und das mindestens eine Ion unter Verwendung von zwei oder mehreren Kollisionsenergien fragmentiert, wodurch die Vielzahl von erfassten Fragmentionenspektren erzeugt wird;
Berechnen von einer oder mehreren Probefragmentionenraten der Veränderung von Massenintensität in Bezug auf Kollisionsenergie für ein oder mehrere Probefragmentionen aus der Vielzahl von erfassten Fragmentionenspektren;
Berechnen von einer oder mehreren Datenbankfragmentionenraten der Veränderung von Massenintensität in Bezug auf Kollisionsenergie für ein oder mehrere Datenbankfragmentionen aus einer oder mehreren bekannten Verbindungen in der Datenbank, wobei die Datenbank aus bekannten Verbindungen für jedes Fragmention von jeder bekannten Verbindung eine Vielzahl von Fragmentionenspektren beinhaltet, die eine Funktion der Kollisionsenergie sind; und
Vergleichen (320) der einen oder mehreren Probefragmentionenraten der Veränderung von Massenintensität mit der einen oder den mehreren Datenbankfragmentionenraten der Veränderung von Massenintensität, um das mindestens eine Ion zu identifizieren.

8. Verfahren (300) nach Anspruch 7, wobei das Massenspektrometer die Probe unter Verwendung von Tandem-Massenspektrometrie oder Massenspektrometrie/Massenspektrometrie (MS/MS) analysiert.

9. Verfahren (300) nach Anspruch 7, wobei das Massenspektrometer die Probe unter Verwendung von Massenspektrometrie/Massenspektrometrie/Massenspektrometrie (MS³) analysiert.

10. Verfahren (300) nach einem der Ansprüche 7 bis 9, wobei das Vergleichen der einen oder mehreren Probefragmentionenraten der Veränderung mit der einen oder den mehreren Datenbankfragmentionenraten der Veränderung Folgendes umfasst
Vergleichen von einer oder mehreren erfassten Kurven von Massenintensität im Vergleich zu Kollisionsenergie, berechnet für ein oder mehrere erfasste Fragmentionen aus der Vielzahl von erfassten Framentionenspektren mit einer oder mehreren bekannten Kurven von Massenintensität im Vergleich zu Kollisionsenergie, berechnet für ein oder mehrere gespeicherte Fragmentionen von einer oder mehreren bekannten Verbindungen in der Datenbank.

11. Verfahren (300) nach einem der Ansprüche 7 bis 9, wobei das Vergleichen der einen oder mehreren Probefragmentionenraten der Veränderung mit der einen oder den mehreren Datenbankfragmentionenraten der Veränderung Folgendes umfasst
Vergleichen von einem oder mehreren erfassten Bildern, berechnet für ein oder mehrere erfasste Fragmentionen aus der Vielzahl von erfassten Fragmentionenspektren mit einem oder mehreren bekannten Bildern, berechnet für ein oder mehrere gespeicherte Fragmentionen von einer oder mehreren bekannten Verbindungen in der Datenbank.

12. Verfahren (300) nach Anspruch 11, wobei jedes Element von jedem Bild aus dem einen oder den mehreren Probefragmentionenbildern eine Kombination aus Masse, Massenintensität und Kollisionsenergie (CE) ist, und jedes Element von jedem Bild aus dem einen oder den mehreren Datenbankfragmentionenbildern eine Kombination aus Masse, Massenintensität und Kollisionsenergie (CE) ist.

13. Computerprogrammprodukt, umfassend ein nichttransitorisches und materielles computerlesbares Speichermedium, dessen Inhalt ein Programm mit Anweisungen beinhaltet, das auf einem Prozessor ausgeführt wird, um ein Verfahren zur Verbindungsidentifikation unter Verwendung von mehreren Spektren, die eine Funktion von Kollisionsenergie sind, durchzuführen, wobei das Verfahren Folgendes umfasst:
Bereitstellen eines Systems (400), wobei das System ein oder mehrere verschiedene Softwaremodule umfasst, und wobei die verschiedenen Softwaremodule ein Messmodul (410) und ein Analysemodul (420) umfassen;
Empfangen (310) einer Vielzahl von erfassten Fragmentionenspektren, die eine Funktion von Kollisionsenergie sind, für mindestens ein Ion aus einem Massenspektrometer, unter Verwendung des Messmoduls, wobei das Massenspektrometer eine Probe analysiert und in jedem Zyklus des Massenspektrometers das mindestens eine Ion auswählt und das mindestens eine Ion unter Verwendung von zwei oder mehreren Kollisionsenergien fragmentiert, wodurch die Vielzahl von erfassten Fragmentionenspektren erzeugt wird;
Berechnen von einer oder mehreren Probefragmentionenraten der Veränderung von Massenintensität in Bezug auf Kollisionsenergie für ein oder mehrere Probefragmentionen aus der Vielzahl von erfassten Fragmentionenspektren;
Berechnen von einer oder mehreren Datenbankfragmentionenraten der Veränderung von Massenintensität in Bezug auf Kollisionsenergie für ein oder mehrere Datenbankfragmentionen aus einer oder mehreren bekannten Verbindungen in der Datenbank, wobei die Datenbank aus bekannten Verbindungen für jedes Fragmention von jeder bekannten Verbindung eine Vielzahl von Fragmentionenspektren beinhaltet, die eine Funktion der Kollisionsenergie sind; und
Vergleichen (320) der einen oder mehreren Probefragmentionenraten der Veränderung von Massenintensität mit der einen oder den mehreren Datenbankfragmentionenraten der Veränderung von Massenintensität, um das mindestens eine Ion zu identifizieren.

## Revendications

1. Système (200) pour l'identification de composés à l'aide de multiples spectres qui sont une fonction d'énergie de collision, comprenant :
un spectromètre de masse (210) qui est configuré pour analyser un échantillon et dans chaque cycle du spectromètre de masse pour sélectionner au moins un ion et pour fragmenter l'au moins un ion à l'aide de deux énergies de collision ou plus, ce qui produit une pluralité de spectres d'ion fragment acquis qui sont une fonction d'énergie de collision ;
une base de données (230) de composés connus qui comporte pour chaque composé connu une pluralité de spectres d'ion fragment qui sont une fonction d'énergie de collision ; et
un processeur (220) en communication avec le spectromètre de masse et la base de données qui est configuré pour :
recevoir à partir du spectromètre de masse la pluralité de spectres d'ion fragment acquis pour l'au moins un ion,
calculer un ou plusieurs taux de variation d'intensité de masse d'ion fragment d'échantillon, vis-à-vis de l'énergie de collision, pour un ou plusieurs ions fragments d'échantillon à partir de la pluralité de spectres d'ion fragment acquis
calculer un ou plusieurs taux de variation d'intensité de masse d'ion fragment de base de données, vis-à-vis de l'énergie de collision, pour un ou plusieurs ions fragments de base de données d'un ou de plusieurs composés connus dans la base de données, et
comparer les un ou plusieurs taux de variation d'intensité de masse d'ion fragment d'échantillon avec les un ou plusieurs taux de variation d'intensité de masse d'ion fragment de base de données afin d'identifier l'au moins un ion.

2. Système (200) selon la revendication 1, dans lequel le spectromètre de masse (210) est configuré pour analyser l'échantillon à l'aide d'une spectrométrie de masse en tandem, ou d'une spectrométrie de masse/spectrométrie de masse (MS/MS).

3. Système (200) selon la revendication 1, dans lequel le spectromètre de masse (210) est configuré pour analyser l'échantillon à l'aide d'une spectrométrie de masse/spectrométrie de masse/spectrométrie de masse (MS³).

4. Système (200) selon l'une quelconque des revendications 1 à 3, dans lequel la comparaison des un ou plusieurs taux de variation d'ion fragment d'échantillon avec les un ou plusieurs taux de variation d'ion fragment de base de données comprend
la comparaison d'une ou de plusieurs courbes d'intensité de masse acquises par rapport à l'énergie de collision calculée pour un ou plusieurs ions fragments acquis de la pluralité de spectres d'ion fragment acquis avec une ou plusieurs courbes d'intensité de masse connues par rapport à l'énergie de collision calculées pour un ou plusieurs ions fragments stockés d'un ou de plusieurs composés connus dans la base de données.

5. Système (200) selon l'une quelconque des revendications 1 à 3, dans lequel la comparaison des un ou de plusieurs taux de variation d'ion fragment d'échantillon avec les un ou plusieurs taux de variation d'ion fragment de base de données comprend
la comparaison d'une ou de plusieurs images acquises calculées pour un ou plusieurs ions fragments acquis à partir de la pluralité de spectres d'ion fragment acquis avec une ou plusieurs images connues calculées pour un ou plusieurs ions fragments stockés d'un ou de plusieurs composés connus dans la base de données.

6. Système (200) selon la revendication 5, dans lequel chaque élément de chaque image des une ou plusieurs images acquises est une combinaison de masse, d'intensité de masse, et d'énergie de collision (CE) et chaque élément de chaque image des une ou plusieurs images connues est une combinaison de masse, d'intensité de masse, et d'énergie de collision (CE).

7. Procédé (300) pour l'identification de composés à l'aide de multiples spectres qui sont une fonction d'énergie de collision, comprenant :
la réception (310) à partir d'un spectromètre de masse d'une pluralité de spectres d'ion fragment acquis qui sont une fonction d'énergie de collision pour au moins un ion à l'aide d'un processeur, dans lequel le spectromètre de masse analyse un échantillon et dans chaque cycle du spectromètre de masse sélectionne l'au moins un ion et fragmente l'au moins un ion à l'aide de deux énergies de collision ou plus, ce qui produit la pluralité de spectres d'ion fragment acquis ;
le calcul d'un ou de plusieurs taux de variation d'intensité de masse d'ion fragment d'échantillon, vis-à-vis de l'énergie de collision, pour un ou plusieurs ions fragments d'échantillon à partir de la pluralité de spectres d'ion fragment acquis ;
le calcul d'un ou de plusieurs taux de variation d'intensité de masse d'ion fragment de base de données, vis-à-vis de l'énergie de collision, pour un ou plusieurs ions fragments de base de données d'un ou de plusieurs composés connus dans la base de données, dans lequel la base de données de composés connus comporte pour chaque ion fragment de chaque composé connu une pluralité de spectres d'ion fragment qui sont une fonction de l'énergie de collision ; et
la comparaison (320) des un ou plusieurs taux de variation d'intensité de masse d'ion fragment d'échantillon avec les un ou plusieurs taux de variation d'intensité de masse d'ion fragment de base de données afin d'identifier l'au moins un ion.

8. Procédé (300) selon la revendication 7, dans lequel le spectromètre de masse (210) analyse l'échantillon à l'aide d'une spectrométrie de masse en tandem, ou d'une spectrométrie de masse/spectrométrie de masse (MS/MS).

9. Procédé (300) selon la revendication 7, dans lequel le spectromètre de masse (210) analyse l'échantillon à l'aide d'une spectrométrie de masse/spectrométrie de masse/spectrométrie de masse (MS³).

10. Procédé (300) selon l'une quelconque des revendications 7 à 9, dans lequel la comparaison des un ou plusieurs taux de variation d'ion fragment d'échantillon avec les un ou plusieurs taux de variation d'ion fragment de base de données comprend
la comparaison d'une ou de plusieurs courbes d'intensité de masse acquises par rapport à l'énergie de collision calculées pour un ou plusieurs ions fragments acquis de la pluralité de spectres d'ion fragment acquis avec une ou plusieurs courbes d'intensité de masse connues par rapport à l'énergie de collision calculées pour un ou plusieurs ions fragments stockés d'un ou de plusieurs composés connus dans la base de données.

11. Procédé (300) selon l'une quelconque des revendications 7 à 9, dans lequel la comparaison des un ou de plusieurs taux de variation d'ion fragment d'échantillon avec les un ou plusieurs taux de variation d'ion fragment de base de données comprend
la comparaison d'une ou de plusieurs images acquises calculées pour un ou plusieurs ions fragments acquis de la pluralité de spectres d'ion fragment acquis avec une ou plusieurs images connues calculées pour un ou plusieurs ions fragments stockés d'un ou de plusieurs composés connus dans la base de données.

12. Procédé (300) selon la revendication 11, dans lequel chaque élément de chaque image des une ou plusieurs images d'ion fragment d'échantillon est une combinaison de masse, d'intensité de masse, et d'énergie de collision (CE) et chaque élément de chaque image des une ou plusieurs images d'ion fragment de base de données est une combinaison de masse, d'intensité de masse, et d'énergie de collision (CE).

13. Produit de programme d'ordinateur, comprenant un support de stockage non transitoire et tangible lisible par ordinateur dont le contenu comporte un programme avec des instructions qui sont exécutées sur un processeur de manière à réaliser un procédé pour l'identification de composés à l'aide de multiples spectres qui sont une fonction d'énergie de collision, le procédé comprenant :
la fourniture d'un système (400), dans lequel le système comprend un ou plusieurs modules logiciels distincts, et dans lequel les modules logiciels distincts comprennent un module de mesure (410) et un module d'analyse (420) ;
la réception (310) à partir d'un spectromètre de masse d'une pluralité de spectres d'ion fragment acquis qui sont une fonction d'énergie de collision pour au moins un ion à l'aide du module de mesure, dans lequel le spectromètre de masse analyse un échantillon et dans chaque cycle du spectromètre de masse sélectionne l'au moins un ion et fragmente l'au moins un ion à l'aide de deux énergies de collision ou plus, ce qui produit la pluralité de spectres d'ion fragment acquis ;
le calcul d'un ou de plusieurs taux de variation d'intensité de masse d'ion fragment d'échantillon, vis-à-vis de l'énergie de collision, pour un ou plusieurs ions fragments d'échantillon à partir de la pluralité de spectres d'ion fragment acquis ;
le calcul d'un ou de plusieurs taux de variation d'intensité de masse d'ion fragment de base de données, vis-à-vis de l'énergie de collision, pour un ou plusieurs ions fragments de base de données d'un ou de plusieurs composés connus dans la base de données, dans lequel la base de données de composés connus comporte pour chaque ion fragment de chaque composé connu une pluralité de spectres d'ion fragment qui sont une fonction d'énergie de collision ; et
la comparaison (320) des un ou plusieurs taux de variation d'intensité de masse d'ion fragment d'échantillon avec les ou plusieurs taux de variation d'intensité de masse d'ion fragment de base de données afin d'identifier l'au moins un ion.
